# EUROPEAN PATENT APPLICATION

(11) **EP 3 363 442 A1**
(43) Date of publication of application: **22.08.2018**
(21) Application number: 16855442.6
(22) Date of filing: 13.10.2016
(51) Int. Cl.: A61K 31/715, A61K 31/723, A61K 31/726, A61K 31/728, A61K 31/737, A61K 45/00, A61K 47/02, A61K 47/10, A61K 47/12, A61K 47/34, A61P 1/00

(54) **PROTECTIVE COMPOSITION FOR GASTROINTESTINAL MUCOSA**

(30) Priority: 13.10.2015 JP 2015202493
(71) Applicant: Techno Guard Co., Ltd., Kanagawa 211-0006 (JP)
(72) Inventor: NABETA Kiichiro, Kawasaki-shi Kanagawa 211-0006 (JP)
(74) Representative: Louis Pöhlau Lohrentz
(86) International application number: PCT/JP2016/080288
(87) International publication number: WO 2017/065189

(57) **Abstract**

An object of the present invention is to provide a protective composition for gastrointestinal mucosa, for example, for inhibiting occurrence of a gastrointestinal mucosa disorder when an oral drug that induces the gastrointestinal mucosa disorder is taken. The protective composition for gastrointestinal mucosa of the present invention as a resolution for achieving the object is characterized by comprising at least a macromolecular polysaccharide, sodium hydrogen carbonate and/or magnesium carbonate, and a polyhydric alcohol. According to the protective composition for gastrointestinal mucosa of the present invention, sodium hydrogen carbonate or magnesium carbonate foams through contact with a gastric juice in stomach to thereby generate diffusing power for the macromolecular polysaccharide. This power can allow the macromolecular polysaccharide to quickly dissolve or disperse in the gastric juice and allow the macromolecular polysaccharide to effectively exert a gastric mucosa protective action. In addition, the macromolecular polysaccharide dissolved or dispersed in the gastric juice moves into intestine without degradation in stomach, then dissolves in an intestinal juice having a neutral or alkaline pH, and exerts an intestinal mucosa protective action without degradation also in intestine. The polyhydric alcohol plays a role as a dispersion medium for the macromolecular polysaccharide and sodium hydrogen carbonate or magnesium carbonate which are powder, or enables formulation of the protective composition for gastrointestinal mucosa of the present invention into various dosage forms, according to the nature and use amount thereof.

## Description

### Technical Field

The present invention relates to a protective composition for gastrointestinal mucosa, for example, for inhibiting occurrence of a gastrointestinal mucosa disorder when an oral drug that induces the gastrointestinal mucosa disorder is taken.

### Background Art

Some of oral drugs induce a gastrointestinal mucosa disorder as an adverse effect on the other side of their excellent pharmacological function, and nonsteroidal anti-inflammatory drugs (NSAIDs) are a typical example thereof. Thus, a method is demanded for inhibiting occurrence of a gastrointestinal mucosa disorder due to an oral drug that induces the gastrointestinal mucosa disorder by protecting gastrointestinal mucosa when the drug is taken.

As an ingredient having a protective action for gastrointestinal mucosa, various ingredients have been heretofore reported, and one example is a macromolecular polysaccharide such as a mucopolysaccharide typified by hyaluronic acid. For example, Patent Document 1 proposes a gastric mucosa protective agent containing chondroitin sulfate which is one of mucopolysaccharides.

### Prior Art Documents

### Patent Document

Patent Document 1: JP-A-5-320056

### Summary of the Invention

### Problems that the Invention is to Solve

In the case of using a macromolecular polysaccharide as an ingredient for inhibiting occurrence of a gastrointestinal mucosa disorder when an oral drug that induces the gastrointestinal mucosa disorder is taken, the macromolecular polysaccharide may be taken before or at the same time as the taking of the drug. However, since the macromolecular polysaccharide which is powder has poor solubility in an acidic (pH 1-2) gastric juice, when the macromolecular polysaccharide is taken as it is in the powder form, the macromolecular polysaccharide can not quickly dissolve or disperse in stomach to exert the mucosa protection effect. Although there is not such a problem when the macromolecular polysaccharide is taken in an aqueous solution form, when the macromolecular polysaccharide is tried to be dissolved in water for preparing an aqueous solution of the macromolecular polysaccharide, an undissolved lump is generated and thus it is taken a long period of time to dissolve the lump. Even if the lump can be dissolved over a long period of time, the viscosity of the aqueous solution may increase or the macromolecular polysaccharide in water may degrade with time. Accordingly, it is impossible to prepare or formulate an aqueous solution of a macromolecular polysaccharide at time of use.

Accordingly, the present invention has an object to provide a protective composition for gastrointestinal mucosa containing a macromolecular polysaccharide, for example, for inhibiting occurrence of a gastrointestinal mucosa disorder when an oral drug that induces the gastrointestinal mucosa disorder is taken.

### Means for Solving the Problems

A protective composition for gastrointestinal mucosa of the present invention made in view of the above point is characterized, as set forth in claim 1, by comprising at least a macromolecular polysaccharide, sodium hydrogen carbonate and/or magnesium carbonate, and a polyhydric alcohol.

The protective composition for gastrointestinal mucosa set forth in claim 2 is characterized, in the protective composition for gastrointestinal mucosa according to claim 1, in that the macromolecular polysaccharide is at least one selected from a mucopolysaccharide, xanthan gum, andfucoidan.

The protective composition for gastrointestinal mucosa set forth in claim 3 is characterized, in the protective composition for gastrointestinal mucosa according to claim 1, in that the polyhydric alcohol is at least one selected from glycerine, diglycerine, polyglycerine, polyethylene glycol, propylene glycol, dipropylene glycol, polypropylene glycol, isopropylene glycol, and 1,3-butylene glycol.

The protective composition for gastrointestinal mucosa set forth in claim 4 is characterized, in the protective composition for gastrointestinal mucosa according to claim 1, by further comprising a polyvalent carboxylic acid or a pharmaceutically acceptable salt thereof.

The protective composition for gastrointestinal mucosa set forth in claim 5 is characterized, in the protective composition for gastrointestinal mucosa according to claim 1, by further comprising an oral drug.

Furthermore, an oral formulation of the present invention is characterized, as set forth in claim 6, in that the protective composition for gastrointestinal mucosa according to claim 1 is formulated.

### Effect of the Invention

According to the protective composition for gastrointestinal mucosa of the present invention, sodium hydrogen carbonate or magnesium carbonate foams through contact with a gastric juice in stomach to thereby generate diffusing power for the macromolecular polysaccharide. This power can allow the macromolecular polysaccharide to quickly dissolve or disperse in the gastric juice and allow the macromolecular polysaccharide to effectively exert a gastric mucosa protective action. In addition, the macromolecular polysaccharide dissolved or dispersed in the gastric juice moves into intestine without degradation in stomach, then dissolves in an intestinal juice having a neutral or alkaline pH (6-8), and exerts an intestinal mucosa protective action without degradation also in intestine. The polyhydric alcohol plays a role as a dispersion medium for the macromolecular polysaccharide and sodium hydrogen carbonate or magnesium carbonate which are powder, or enables formulation of the protective composition for gastrointestinal mucosa of the present invention into various dosage forms, according to the nature and the use amount thereof.

### Brief Description of the Drawings

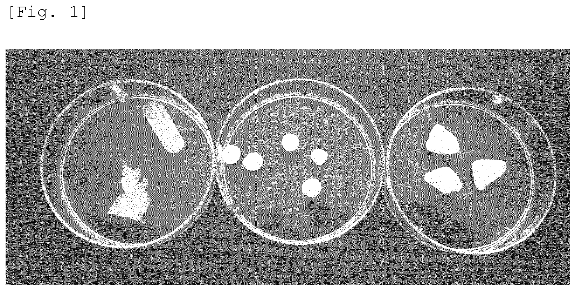

Fig. 1 shows appearances of the protective composition for gastrointestinal mucosa of the present invention. The left dish shows a composition of a gel form obtained in Example 1 (the lower is the composition itself and the upper is the composition filled in a hard capsule) . The central dish shows a composition of a clay form obtained in Example 2. The right dish shows a composition of a solid form obtained in Example 3.

### Means for Carrying Out the Invention

A protective composition for gastrointestinal mucosa of the present invention is characterized by containing at least a macromolecular polysaccharide, sodium hydrogen carbonate and/or magnesium carbonate, and a polyhydric alcohol.

The macromolecular polysaccharide contained in the protective composition for gastrointestinal mucosa of the present invention may be, for example, one that is known to have a protective action for gastrointestinal mucosa, and specific examples thereof include a mucopolysaccharide, xanthan gum, and fucoidan. Mucopolysaccharides are a macromolecular polysaccharide having an aminosugar or a derivative thereof as a constituent sugar, and the mucopolysaccharide may be an acidic mucopolysaccharide or a neutral mucopolysaccharide. As an acidic mucopolysaccharide, hyaluronic acid, chondroitin sulfate A, chondroitin sulfate C, dermatan sulfate (chondroitin sulfate B), heparin, keratan sulfate, etc. are exemplified. As a neutral mucopolysaccharide, chitin and chitosan are exemplified. Xanthan gum is a macromolecular polysaccharide obtained by microbial fermentation. Fucoidan is a macromolecular polysaccharide largely contained in mucilage such as kombu seaweed, wakame seaweed, and mozuku seaweed. As a macromolecular polysaccharide, for example, commercially available powder having a molecular weight of 100,000 to 5,000,000 may be used. When the molecular weight of the macromolecular polysaccharide is lower than 100,000, the protective action for gastrointestinal mucosa is deteriorated. On the other hand, when the molecular weight exceeds 5,000,000, the macromolecular polysaccharide is difficult to dissolve or disperse in a gastric juice. The molecular weight of the macromolecular polysaccharide is desirably 500,000 to 3,000,000.

Sodium hydrogen carbonate or magnesium carbonate contained in the protective composition for gastrointestinal mucosa of the present invention foams through contact with a gastric juice in stomach to generate diffusing power for the macromolecular polysaccharide, the power allowing the macromolecular polysaccharide to quickly dissolve or disperse in the gastric juice. Either one of sodium hydrogen carbonate and magnesium carbonate may be used or both may be used in mixture.

Specific examples of the polyhydric alcohol contained in the protective composition for gastrointestinal mucosa of the present invention include glycerine, diglycerine, polyglycerine, polyethylene glycol (macrogol), propylene glycol, dipropylene glycol, polypropylene glycol, isopropylene glycol, and 1,3-butylene glycol. Average molecular weights of polyglycerine, polyethylene glycol, and polypropylene glycol each may be, for example, 200 to 35,000. The polyhydric alcohol may be used alone or in mixture of plural kinds, taking the nature and the use amount into account.

In the protective composition for gastrointestinal mucosa of the present invention, in order to allow sodium hydrogen carbonate or magnesium carbonate to foam through contact with s gastric juice to generate diffusing power for the macromolecular polysaccharide and to allow the macromolecular polysaccharide to dissolve or disperse in the gastric juice by the power, the mixing ratio of sodium hydrogen carbonate or magnesium carbonate to the macromolecular polysaccharide is desirably 0.1 g to 50 g, more desirably 0.5 g to 30 g, and further desirably 1 g to 15 g relative to 1 g of the macromolecular polysaccharide. Incidentally, the dose of the macromolecular polysaccharide at one time by the protective composition for gastrointestinal mucosa of the present invention is desirably 10 mg to 500 mg for allowing the macromolecular polysaccharide to effectively exert the protective action for the gastrointestinal mucosa (the protective composition for gastrointestinal mucosa of the present invention may be formulated so that one formulation contains the above amount of the macromolecular polysaccharide).

The protective composition for gastrointestinal mucosa of the present invention can be produced, for example, by mixing a macromolecular polysaccharide, sodium hydrogen carbonate or magnesium carbonate, and a polyhydric alcohol. The polyhydric alcohol may be mixed with the macromolecular polysaccharide and sodium hydrogen carbonate or magnesium carbonate, as it is if the polyhydric alcohol is in a liquid form at a normal temperature (for example, 25°C), or after heating if the polyhydric alcohol is in a solid form at the above temperature. The content of the polyhydric alcohol in the protective composition for gastrointestinal mucosa of the present invention may be, for example, 5% to 90% relative to the total weight of the composition. Although depending on the kind of the polyhydric alcohol, when the content of the polyhydric alcohol is, for example, 10% or more relative to the total weight of the composition, the polyhydric alcohol plays a role as a dispersion medium for the macromolecular polysaccharide and sodium hydrogen carbonate or magnesium carbonate which are powder. Since the macromolecular polysaccharide and sodium hydrogen carbonate or magnesium carbonate can not dissolve in a polyhydric alcohol, these substances are stably present in the polyhydric alcohol as it is in a powder form. By using a polyhydric alcohol alone, taking the nature into account, or using plural polyhydric alcohols having different natures in mixture of a prescribed ratio, the protective composition for gastrointestinal mucosa of the present invention can be obtained in different forms such as a liquid form, a gel form, a semi-solid form, and a solid form in which the macromolecular polysaccharide and sodium hydrogen carbonate or magnesium carbonate are dispersed in the polyhydric alcohol, whereby the composition can be formulated into various dosage forms. In any dosage form, after taking, the polyhydric alcohol quickly dissolves in a gastric juice in stomach, whereby sodium hydrogen carbonate or magnesium carbonate foams through contact with the gastric juice to thereby generate diffusing power for the macromolecular polysaccharide, the power then allowing the macromolecular polysaccharide to quickly dissolve or disperse in the gastric juice and allowing the macromolecular polysaccharide to effectively exert a gastric mucosa protective action (when the volume of the gastric acid is increased by water taken at the taking of the composition, the effect increases) . In addition, the macromolecular polysaccharide dissolved or dispersed in the gastric juice moves into intestine without degradation in stomach, then dissolves in an intestinal juice having a neutral or alkaline pH, and exerts an intestinal mucosa protective action without degradation also in intestine.

In addition, although depending on the kind of the polyhydric alcohol, when the content of the polyhydric alcohol is, for example, 20% or less relative to the total weight of the composition, the polyhydric alcohol plays a role as a binder between the macromolecular polysaccharide and sodium hydrogen carbonate or magnesium carbonate, whereby the protective composition for gastrointestinal mucosa of the present invention can be formulated into a solid form. Also in this composition, sodium hydrogen carbonate or magnesium carbonate foams through contact with a gastric juice in stomach to generate diffusing power for the macromolecular polysaccharide, the power then allowing the macromolecular polysaccharide to quickly dissolve or disperse in the gastric juice and allowing the macromolecular polysaccharide to effectively exert a gastric mucosa protective action (when the volume of the gastric acid is increased by water taken at the taking of the composition, the effect increases) . In addition, the macromolecular polysaccharide dissolved or dispersed in the gastric juice moves into intestine without degradation in stomach, then dissolves in an intestinal juice having a neutral or alkaline pH, and exerts an intestinal mucosa protective action without degradation also in intestine.

The protective composition for gastrointestinal mucosa of the present invention may further contain a polyvalent carboxylic acid or a pharmaceutically acceptable salt thereof. When such a substance is contained, even if the pH in stomach does not have an enough acidity to allow sodium hydrogen carbonate or magnesium carbonate to foam (for example, when the pH exceeds 3), the pH can be lowered to induce the sodium hydrogen carbonate or magnesium carbonate to foam. Specific examples of the polyvalent carboxylic acid include citric acid, succinic acid, maleic acid, fumaric acid, malic acid, and tartaric acid. Examples of the pharmaceutically acceptable salt of a polyvalent carboxylic acid include a sodium salt, a potassium salt, a calcium salt, and an ammonium salt. When a free polyvalent carboxylic acid has or may have an adverse effect on stability of another component contained in the protective composition for gastrointestinal mucosa of the present invention, not a free polyvalent carboxylic acid but a pharmaceutically acceptable salt of the polyvalent carboxylic acid is desirably used. The content of a polyvalent carboxylic acid or a pharmaceutically acceptable salt thereof may be, for example, 1% to 30% relative to the total weight of the composition.

Incidentally, the protective composition for gastrointestinal mucosa of the present invention may contain, as another component, a cellulose such as crystalline cellulose, carmellose sodium, and hydroxypropyl cellulose, glucose, sorbitol, mannitol, dextrin, potato starch, corn starch, calcium hydrogen phosphate, light anhydrous silicic acid, etc. as a binder or an excipient. However, it is desired that substantially no water is contained. The reason is that water, if present in the composition, has an adverse effect on stability of the macromolecular polysaccharide and sodium hydrogen carbonate or magnesium carbonate.

The explanation above is made, for example, about the protective composition for gastrointestinal mucosa of the present invention in which a macromolecular polysaccharide exerts a protective action for gastrointestinal mucosa when the composition is taken before or at the same time as the taking of an oral drug that induces a gastrointestinal mucosa disorder, but the protective composition for gastrointestinal mucosa of the present invention may further contain, for example, an oral drug that induces a gastrointestinal mucosa disorder. The protective composition for gastrointestinal mucosa of the present invention containing an oral drug that induces a gastrointestinal mucosa disorder can be orally administered as a drug composition having a gastrointestinal mucosa protective action. As the oral drug that induces a gastrointestinal mucosa disorder here, exemplified are a nonsteroidal anti-inflammatory drug including diclofenac, indomethacin, ibuprofen, ketoprofen, naproxen, loxoprofen, piroxicam, lornoxicam, and meloxicam; an antipyretic analgesic, such as aspirin, salicylic acid, and acetaminophen; an immunosuppressive drug, such as mizoribine; a calcitonin-related formulation, such as disodium etidronate, ipriflavone, sodium alendronate hydrate, sodium risedronate hydrate, and sevelamer hydrochloride; an antibiotic, such as erythromycin, clindamycin, and ribavirin; a corticosteroid, such as prednisolone, dexamethasone, betamethasone, hydrocortisone, and methylprednisolone; and a lipid-lowering drug, such as probucol (these substances may be in a form of a pharmaceutically acceptable salt thereof). Such an oral drug is not deteriorated in the stability even when existing with a macromolecular polysaccharide, sodium hydrogen carbonate or magnesium carbonate, and a polyhydric alcohol, and thus stably exists in the composition. Incidentally, in the protective composition for gastrointestinal mucosa of the present invention containing an oral drug that induces a gastrointestinal mucosa disorder, the content of the oral drug can be appropriately determined so that the oral drug exerts a prescribed pharmacological action when a formulated composition is taken (the content of the oral drug in one formulation is, for example, 1 mg to 3 g).

### Examples

Hereinunder, the present invention is explained in detail with reference to examples. The present invention should not be construed to be limited to the following description.

### Example 1:

A protective composition for gastrointestinal mucosa of the present invention containing powdery ibuprofen sodium salt as an oral drug was obtained in the following manner: 3000 mg of macrogol 400 and 400 mg of macrogol 4000 were uniformly dissolved through heating to 70°C, followed by lowering the liquid temperature to 60°C, then 2000 mg of ibuprofen sodium salt, 150 mg of hyaluronic acid (manufactured by Kewpie Corporation, molecular weight: about 1,200,000, the same applies hereinafter), and 500 mg of sodium hydrogen carbonate were sequentially added, and the mixture was cooled with mixing by thorough stirring. The resulting protective composition for gastrointestinal mucosa of the present invention was a white gel form having high viscosity in which ibuprofen sodium salt, hyaluronic acid, and sodium hydrogen carbonate were uniformly dispersed in a mixture of macrogol 400 and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface. The appearance of the resulting protective composition for gastrointestinal mucosa of the present invention is shown in Fig. 1 (left dish: the lower is the composition itself and the upper is the composition filled in a No. 1 hard capsule made of pullulan, the total content of macrogol 400 and macrogol 4000 was 56% relative to the total weight of the composition) .

### Example 2:

A protective composition for gastrointestinal mucosa of the present invention containing powdery ibuprofen sodium salt as an oral drug was obtained in the following manner: 1000 mg of macrogol 400 and 200 mg of macrogol 4000 were uniformly dissolved through heating to 70°C, followed by lowering the liquid temperature to 60°C, then 2000 mg of ibuprofen sodium salt, 150 mg of hyaluronic acid, and 1000 mg of sodium hydrogen carbonate were sequentially added, and the mixture was cooled with mixing by thorough stirring. The resulting protective composition for gastrointestinal mucosa of the present invention was a white semi-solid form (clay form) in which ibuprofen sodium salt, hyaluronic acid, and sodium hydrogen carbonate were uniformly dispersed by a mixture of macrogol 400 and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface. The appearance of the resulting protective composition for gastrointestinal mucosa of the present invention is shown in Fig. 1 (central dish: the composition formed into a pill form, the total content of macrogol 400 and macrogol 4000 was 28% relative to the total weight of the composition).

### Example 3:

A protective composition for gastrointestinal mucosa of the present invention containing powdery naproxen sodium salt as an oral drug was obtained in the following manner: 800 mg of macrogol 400 and 100 mg of macrogol 4000 were uniformly dissolved through heating to 70°C, followed by lowering the liquid temperature to 60°C, then 2000 mg of naproxen sodium salt, 150 mg of hyaluronic acid, and 2000 mg of sodium hydrogen carbonate were sequentially added, and the mixture was cooled with mixing by thorough stirring. The resulting protective composition for gastrointestinal mucosa of the present invention was a white semi-solid form (clay form) in which naproxen sodium salt, hyaluronic acid, and sodium hydrogen carbonate were uniformly dispersed by a mixture of macrogol 400 and macrogol 4000. When allowed to stand in a room for one day, the composition was changed into a solid, and when put into a pharmacopeial artificial gastric juice, the composition immediately foamed vigorously and dispersed therein to form a uniform white opaque liquid, and then the components of the composition became dissolved or dispersed in the liquid without remaining on the liquid surface. The appearance of the resulting protective composition for gastrointestinal mucosa of the present invention (solid) is shown in Fig. 1 (right dish, the total content of macrogol 400 and macrogol 4000 is 18% relative to the total weight of the composition).

### Example 4:

A protective composition for gastrointestinal mucosa of the present invention containing powdery diclofenac sodium salt as an oral drug was obtained in the following manner: 2000 mg of macrogol 400 and 320 mg of macrogol 4000 were uniformly dissolved through heating to 65°C, then 200 mg of diclofenac sodium salt, 120 mg of hyaluronic acid, and 400 mg of sodium hydrogen carbonate were sequentially added, and the mixture was cooled with mixing by thorough stirring. The resulting protective composition for gastrointestinal mucosa of the present invention was a white viscous opaque liquid form in which diclofenac sodium salt, hyaluronic acid, and sodium hydrogen carbonate were uniformly dispersed in a mixture of macrogol 400 and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface.

### Example 5:

A protective composition for gastrointestinal mucosa of the present invention containing powdery diclofenac sodium salt as an oral drug was obtained in the following manner: 2500 mg of macrogol 400 and 400 mg of macrogol 4000 were uniformly dissolved through heating to 65°C, then 250 mg of diclofenac sodium salt, 150 mg of hyaluronic acid, and 500 mg of sodium hydrogen carbonate were sequentially added, and the mixture was cooled with mixing by thorough stirring. The resulting protective composition for gastrointestinal mucosa of the present invention was a white gel form having high viscosity in which diclofenac sodium salt, hyaluronic acid, and sodium hydrogen carbonate were uniformly dispersed in a mixture of macrogol 400 and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface.

### Example 6:

A protective composition for gastrointestinal mucosa of the present invention containing powdery diclofenac sodium salt as an oral drug was obtained in the following manner: 2500 mg of macrogol 400 and 400 mg of macrogol 4000 were uniformly dissolved through heating to 65°C, then 250 mg of diclofenac sodium salt, 150 mg of sodium chondroitin sulfate (manufactured by Kishida Chemical Co., Ltd.), and 500 mg of sodium hydrogen carbonate were sequentially added, and the mixture was cooled with mixing by thorough stirring. The resulting protective composition for gastrointestinal mucosa of the present invention was a white semi-solid form (wax form) in which diclofenac sodium salt, sodium chondroitin sulfate, and sodium hydrogen carbonate were uniformly dispersed by a mixture of macrogol 400 and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface.

### Example 7:

A protective composition for gastrointestinal mucosa of the present invention containing powdery diclofenac sodium salt as an oral drug was obtained in the following manner: 2500 mg of macrogol 400 and 400 mg of macrogol 4000 were uniformly dissolved through heating to 65°C, then 250 mg of diclofenac sodium salt, 150 mg of chitosan (manufactured by Yaizu Suisankagaku Industry Co., Ltd.), and 500 mg of sodium hydrogen carbonate were sequentially added, and the mixture was cooled with mixing by thorough stirring. The resulting protective composition for gastrointestinal mucosa of the present invention was a white gel form having high viscosity in which diclofenac sodium salt, chitosan, and sodium hydrogen carbonate were uniformly dispersed in a mixture of macrogol 400 and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface.

### Example 8:

A protective composition for gastrointestinal mucosa of the present invention containing powdery diclofenac sodium salt as an oral drug was obtained in the following manner: 2500 mg of macrogol 400 and 400 mg of macrogol 4000 were uniformly dissolved through heating to 65°C, then 250 mg of diclofenac sodium salt, 150 mg of fucoidan (manufactured by Yaizu Suisankagaku Industry Co., Ltd., molecular weight: about 200,000), and 500 mg of sodium hydrogen carbonate were sequentially added, and the mixture was cooled with mixing by thorough stirring. The resulting protective composition for gastrointestinal mucosa of the present invention was a white gel form having high viscosity in which diclofenac sodium salt, fucoidan, and sodium hydrogen carbonate were uniformly dispersed in a mixture of macrogol 400 and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface.

### Example 9:

A protective composition for gastrointestinal mucosa of the present invention containing powdery naproxen sodium salt as an oral drug was obtained in the following manner: 1500 mg of propylene glycol and 2150 mg of macrogol 4000 were uniformly dissolved through heating to 70°C, followed by lowering the liquid temperature to 60°C, then 2000 mg of naproxen sodium salt, 150 mg of hyaluronic acid, and 1500 mg of sodium hydrogen carbonate were sequentially added, and the mixture was cooled with mixing by thorough stirring. The resulting protective composition for gastrointestinal mucosa of the present invention was a white semi-solid form (wax form) in which naproxen sodium salt, hyaluronic acid, and sodium hydrogen carbonate were uniformly dispersed by a mixture of propylene glycol and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface.

### Example 10:

A protective composition for gastrointestinal mucosa of the present invention containing powdery diclofenac sodium salt as an oral drug was obtained in the following manner: 500 mg of macrogol 400 and 500 mg of macrogol 4000 were uniformly dissolved through heating to 70°C, followed by lowering the liquid temperature to 60°C, then 250 mg of diclofenac sodium salt, 150 mg of hyaluronic acid, 1500 mg of sodium hydrogen carbonate, and 1500 mg of potato starch were sequentially added, and the mixture was cooled with mixing by thorough stirring. The resulting protective composition for gastrointestinal mucosa of the present invention was a white semi-solid form (clay form) in which diclofenac sodium salt, hyaluronic acid, sodium hydrogen carbonate, and potato starch were uniformly dispersed by a mixture of macrogol 400 and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface.

### Example 11:

A protective composition for gastrointestinal mucosa of the present invention containing powdery prednisolone as an oral drug was obtained in the following manner: to 800 mg of macrogol 400 heated to 65°C were sequentially added 5 mg of prednisolone, 25mg of hyaluronic acid, and 50 mg of sodium hydrogen carbonate, and the mixture was cooled with mixing by thorough stirring. The resulting protective composition for gastrointestinal mucosa of the present invention was a white viscous opaque liquid form in which prednisolone, hyaluronic acid, and sodium hydrogen carbonate were uniformly dispersed in macrogol 400. When put in a pharmacopeial artificial gastric juice, the composition immediately foamed vigorously and dispersed therein to form a uniform white opaque liquid, and then the components of the composition became dissolved or dispersed in the liquid without remaining on the liquid surface.

### Example 12:

A protective composition for gastrointestinal mucosa of the present invention containing no oral drug was obtained in the following manner: 1840 mg of macrogol 400 and 40 mg of macrogol 4000 were uniformly dissolved through heating to 70°C, then 120 mg of hyaluronic acid and 320 mg of sodium hydrogen carbonate were sequentially added, and the mixture was cooled with mixing by thorough stirring. The resulting protective composition for gastrointestinal mucosa of the present invention was a white viscous opaque liquid form in which hyaluronic acid and sodium hydrogen carbonate were uniformly dispersed in a mixture of macrogol 400 and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface.

### Comparative Example 1:

After 1840 mg of macrogol 400 and 40 mg of macrogol 4000 were uniformly dissolved through heating to 70°C, 120 mg of hyaluronic acid was added thereto, and the mixture was cooled with mixing by thorough stirring, whereby a white viscous opaque liquid form in which hyaluronic acid was uniformly dispersed in a mixture of macrogol 400 and macrogol 4000 was obtained. When this liquid was put in a pharmacopeial artificial gastric juice, it only gradually dispersed on a liquid surface, and did not become to a state where hyaluronic acid dissolved or dispersed in the liquid.

### Example 13:

A protective composition for gastrointestinal mucosa of the present invention containing no oral drug was obtained in the following manner: 1840 mg of macrogol 400 and 40 mg of macrogol 4000 were uniformly dissolved through heating to 70°C, then 120 mg of xanthan gum (manufactured by MP Biomedicals, molecular weight: 2,000,000 or higher) and 320 mg of sodium hydrogen carbonate were sequentially added, and the mixture was cooled with mixing by thorough stirring. The resulting protective composition for gastrointestinal mucosa of the present invention was a white viscous opaque liquid form in which xanthan gum and sodium hydrogen carbonate were uniformly dispersed in a mixture of macrogol 400 and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface.

### Example 14:

A protective composition for gastrointestinal mucosa of the present invention containing no oral drug was obtained in the following manner: 1500 mg of polyglycerine 500 and 2150 mg of macrogol 4000 were uniformly dissolved through heating to 70°C, then 150 mg of hyaluronic acid and 1500 mg of magnesium carbonate were sequentially added, and the mixture was cooled with mixing by thorough stirring. The resulting protective composition for gastrointestinal mucosa of the present invention was a white semi-solid form (wax form) in which hyaluronic acid and magnesium carbonate were uniformly dispersed by a mixture of polyglycerine 500 and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface.

### Example 15:

A protective composition for gastrointestinal mucosa of the present invention containing no oral drug was obtained in the following manner: 1500 mg of polyglycerine 500 and 2150 mg of macrogol 4000 were uniformly dissolved through heating to 70°C, then 150 mg of hyaluronic acid, 1500 mg of magnesium carbonate, and 1000 mg of fumaric acid sodium salt were sequentially added, and the mixture was cooled with mixing by thorough stirring. The resulting protective composition for gastrointestinal mucosa of the present invention was a white semi-solid form (wax form) in which hyaluronic acid, magnesium carbonate, and fumaric acid sodium salt were uniformly dispersed by a mixture of polyglycerine 500 and macrogol 4000. When put in a pharmacopeial artificial gastric juice, the composition immediately foamed vigorously and dispersed therein to form a uniform white opaque liquid, and then the components of the composition became dissolved or dispersed in the liquid without remaining on the liquid surface.

### Industrial Availability

The present invention has an industrial availability in the capability of providing a protective composition for gastrointestinal mucosa, for example, for inhibiting occurrence of a gastrointestinal mucosa disorder when an oral drug that induces the gastrointestinal mucosa disorder is taken.

## Claims

1. A protective composition for gastrointestinal mucosa, **characterized by** comprising at least a macromolecular polysaccharide, sodium hydrogen carbonate and/or magnesium carbonate, and a polyhydric alcohol.

2. The protective composition for gastrointestinal mucosa according to claim 1, **characterized in that** the macromolecular polysaccharide is at least one selected from a mucopolysaccharide, xanthan gum, and fucoidan.

3. The protective composition for gastrointestinal mucosa according to claim 1, **characterized in that** the polyhydric alcohol is at least one selected from glycerine, diglycerine, polyglycerine, polyethylene glycol, propylene glycol, dipropylene glycol, polypropylene glycol, isopropylene glycol, and 1,3-butylene glycol.

4. The protective composition for gastrointestinal mucosa according to claim 1, **characterized by** further comprising a polyvalent carboxylic acid or a pharmaceutically acceptable salt thereof.

5. The protective composition for gastrointestinal mucosa according to claim 1, **characterized by** further comprising an oral drug.

6. An oral formulation, **characterized in that** the protective composition for gastrointestinal mucosa according to claim 1 is formulated.
